# EUROPÄISCHE PATENTSCHRIFT

(11) **EP 2 735 279 B1**
(45) Veröffentlichungstag und Bekanntmachung des Hinweises auf die Patenterteilung: **17.04.2019**
(21) Anmeldenummer: 12007906.6
(22) Anmeldetag: 23.11.2012
(51) Int. Cl.: A61C 8/00

(54) **Dentalimplantat**
Dental implant
Implant dentaire

(43) Veröffentlichungstag der Anmeldung: 28.05.2014
(73) Patentinhaber: Lakeview Innovation Ltd., 6374 Buochs (CH)
(72) Erfinder: Buurlage, Thorsten, 69517 Gorxheimertal (DE)
(74) Vertreter: IPrime Rentsch Kaelin AG

(56) Entgegenhaltungen:
- EP-A1- 2 025 303
- WO-A1-2010/106777
- WO-A2-2007/031562
- DE-C1- 19 815 719
- DE-U1-202008 014 591
- FR-A1- 2 945 205

## Beschreibung

Die Erfindung bezieht sich auf ein Dentalimplantat mit einem, in einem Kieferknochen verankerbaren Basiskörper aus Keramik, einem an dem Basiskörper befestigbaren Implantataufbau, und mit einer Schraube zur Befestigung des Implantataufbaus an dem Basiskörper, wobei in einem zusammengebauten Zustand des Dentalimplantats ein Gewindeabschnitt der Schraube in ein, in einem Sackloch des Basiskörpers ausgebildetes Innengewinde eingreift, und wobei der Implantataufbau durch die Schraube gegen den Basiskörper gedrückt ist.

Solche Dentalimplantate sind aus dem Stand der Technik bereits bekannt. Das Anziehen der Schraube führt zu Materialspannungen in Basiskörper und Implantataufbau. Sowohl in den Auflagebereichen zwischen dem Basiskörper und dem Implantataufbau als auch um das Innengewinde herum, in das die Gewindeflanken der Schraube eingreifen, kommt es im angezogenen Zustand der Schraube zu großen Belastungen des verwendeten Keramikmaterials. Insbesondere die für keramische Materialien schädlichen Scher- und Zugbelastungen werden durch die Verschraubung in den Bauteilen generiert. Folglich kommt es somit auch häufig zu Überbeanspruchungen des druckkraftstabilen, jedoch zugkraftinstabilen Keramikmaterials. Beim Anziehen der Schraube kann es im Auflagebereich zwischen dem Basiskörper und dem Implantataufbau zum Anriss oder gar Bruch des Keramikmaterials.

Beispielhaft für diesen Stand der Technik ist die EP 2 168 530 A1, die ein keramisches Implantat offenbart, das in einem Implantatkörper metallische Beschichtungen aufweist, die auf dem Innengewinde aufgebracht sind. Auch an einer Phase, an einem Ende des Basiskörpers, an dem ein Implantataufbau aufliegt, ist eine solche Metallschicht angebracht, um Scher- und Zugbelastungen besser aufnehmen zu können. Solche metallischen Einsätze oder Beschichtungen müssen jedoch durch aufwändige Prozesse auf das Keramikgrundmaterial aufgebracht werden und sind deshalb sehr teuer. Der mit dem Innengewinde in Eingriff befindliche Teil der Schraube ist darüber hinaus im oberen Teil des Basiskörpers angeordnet, während der untere Teil des Basiskörpers als Vollkörper ausgestaltet ist. Somit sind auch die durch das Anziehen der Schraube entstehenden Spannungen auf den oberen Teil des Basiskörpers konzentriert, während im unteren Teil des Implantatkörpers keine Spannungen herrschen. Ein mehrachsiger Spannungszustand umfassend Zug- und Scherkräfte ist in diesem Bauteil somit auf den Auflagebereich zwischen Basiskörper und Implantataufbau und den Bereich um das Innengewinde herum konzentriert. Gleichzeitig sind die Spannungen sehr inhomogen über die gesamte Länge des Basiskörpers verteilt.

Ein Dentalimplantat der eingangs genannten Art ist zudem aus WO 2007/031562 A2 bekannt. Dentalimplantate mit einem Basiskörper und einem Implantataufbau, der mittels einer Schraube an dem Basiskörper befestigt ist, sind ferner auch aus FR 2945205 A1 und aus DE 19815719 C1.

Es ist Aufgabe der vorliegenden Erfindung, ein Dentalimplantat bereitzustellen, das kostengünstig herzustellen ist und eine für Keramikmaterialien optimierte Krafteinleitung aufweist.

Diese Aufgabe wird erfindungsgemäß dadurch gelöst, dass der Eingriff zwischen dem Gewindeabschnitt der Schraube und dem Innengewinde des Loches ausschließlich in einer dem Implantataufbau abgewandten, unteren Hälfte des Basiskörpers besteht, welche untere Hälfte sich über die Hälfte der Länge des Basiskörpers erstreckt, wobei die Sacklochbohrung, an das Innengewinde in Richtung des Implantataufbaus anschließend, als Passbohrung ausgeführt ist, wobei zwischen der Passbohrung und einem Schaft der eingesetzten Schraube eine Spielpassung mit sehr geringem Spiel oder eine Übergangspassung besteht, sodass eine Abstützung in Querrichtung zur Längsachse der Schraube bereitgestellt wird.

Diese Ausgestaltung hat den Effekt, dass die erzeugten Spannungen homogen auf die gesamte Länge des Basiskörpers verteilt werden. Weiterhin liegt das Innengewinde, in das die Spannungen direkt über die Schraube eingeleitet werden, weit von dem Kontaktbereich mit dem Implantataufbau entfernt, tief im Fuß des Basiskörpers. Durch diesen großen Abstand zwischen Auflagebereich und dem mit er Schraube in Eingriff befindlichen Teil des Innengewindes werden in der oberen Hälfte, im Auflagebereich zwischen Basiskörper und Implantataufbau, Zug- und Scherspannungen größtenteils vermieden. Stattdessen liegen in diesem Bereich überwiegend Druckspannungen an, die von dem Keramikmaterial wesentlich besser aufgenommen werden können. Der oft platzbedingt dünnwandige Auflagebereich zwischen Basiskörper und Implantataufbau wird deutlich entlastet und die Einleitung der Schraubenspannung am Innengewinde des Basiskörpers findet in dem massiveren, unteren Teil des Basiskörpers statt. Folglich wird die Rissneigung reduziert und die Montagefreundlichkeit erhöht. Eine zusätzliche Verstärkung des Keramikmaterials wie Metallbeschichtungen im Auflagebereich sowie dem Innengewinde ist somit nicht mehr notwendig, wodurch die Herstellungskosten von Dentalimplantaten wesentlich gesenkt werden. Gar ist es möglich, den Basiskörper und den Implantataufbau vollständig, ohne zusätzliche Beschichtungen, aus Keramik herzustellen, was den Herstellaufwand wiederum reduziert. Zugspannungen treten somit hauptsächlich in der Schraube auf. Hierfür sind besonders gut Schrauben aus Metall geeignet, die wiederum günstig herstellbar sind. Dadurch, dass zwischen der Passbohrung und einem Schaft der eingesetzten Schraube eine Spielpassung mit sehr geringem Spiel oder eine Übergangspassung besteht, wird eine einfache Abstützung in Querrichtung zur Längsachse der Schraube bereitgestellt. Scherkräfte werden somit einfach durch den Schraubenschaft abgestützt.

Vorteilhafte Ausführungsformen sind in den Unteransprüchen beansprucht und nachfolgend erläutert.

So ist es gemäß einer ersten Ausführungsform der Erfindung von Vorteil, wenn sich das Innengewinde über seine gesamte Länge ausschließlich in der unteren Hälfte des Basiskörpers erstreckt. Somit ist es nicht notwendig, ein Gewinde über die gesamte Sacklochlänge des Basiskörpers anzubringen, wodurch der Herstellungsaufwand reduziert wird. Auch Scherspannungen sind dann lediglich auf den unteren Teil beschränkt.

In einer weiteren Ausführungsform der Erfindung ist es zudem vorteilhaft, wenn die Länge des Sackloches mindestens 80 %, bevorzugt mindestens 90 %, der Gesamtlänge des Basiskörpers beträgt. Dadurch wird gesamte Länge des Basiskörpers möglichst effektiv zur Übertragung der Schraubenspannung verwendet.

Von Vorteil ist es auch, wenn gemäß einer weiteren Ausführungsform der Erfindung die Länge der Passbohrung größer als die Länge des Innengewindes ist. Somit ist ein optimales Verhältnis zwischen Seitenabstützung in Querrichtung und Druckentlastung im Kontaktbereich gegeben.

Gemäß einer weiteren Ausführungsform der Erfindung liegt der Implantataufbau an einer im Wesentlichen senkrecht zu der Längsachse der Schraube verlaufenden Auflagefläche an dem Basiskörper an. Das hat den Vorteil, dass die Druckkräfte, die von dem Basiskörper auf den Implantataufbau bzw. umgekehrt übertragen werden, auf eine senkrechte Fläche drücken, wodurch die Belastung durch Scherkräfte vermieden wird. Eine noch keramikfreundlichere Konstruktion ist somit gegeben.

Auch ist von Vorteil, wenn gemäß einer vorteilhaften Ausführungsform der Erfindung der Implantataufbau einen Fortsatz aufweist, der im zusammengebauten Zustand des Dentalimplantats in eine Aussparung des Basiskörpers eingesteckt ist. Somit wird auf einfache Weise eine Lagerung des Implantataufbaus zum Basiskörper, in radialer Richtung, gewährleistet. Die radiale Verschiebung dieser beiden Teile zueinander wird somit vermieden.

Weiterhin ist es gemäß einer vorteilhaften Ausführungsform der Erfindung möglich, zwischen dem Implantataufbau und dem Basiskörper einen verdrehsicheren Formschluss vorzusehen. Insbesondere während des Zusammenschraubens, aber auch während dem Gebrauch wird ein Verdrehen des Implantataufbaus relativ zum Basiskörper vermieden.

Gemäß einer weiteren Ausführungsform der Erfindung ist es von Vorteil, wenn sowohl der Implantataufbau als auch der Basiskörper aus Keramik bestehen, vorzugsweise aus Zirkonoxid. Dies hat den Vorteil, dass eine besonders feste Struktur des Dentalimplantates gewährleistet ist.

Ist gemäß einer weiteren vorteilhaften Ausführungsform der Implantataufbau und/oder der Basiskörper mittels Pulverspritzgießverfahren hergestellt, so wird eine besonders kostengünstige Herstellung des Dentalimplantats ermöglicht. Durch dieses Verfahren kann auch das Innengewinde bereits im Urformprozess mitgeformt werden, was wiederum die Herstellkosten reduziert.

Die Erfindung stellt auch einen Basiskörper für ein Dentalimplantat bereit, der ebenfalls die Vorteile der niedrigen Herstellungskosten und der positiven Spannungsverteilung aufweist, sowie universell auch für andere Implantataufbauten verwendbar ist.

Die Erfindung wird nachfolgend anhand eines Ausführungsbeispiels mit Hilfe von Figuren näher erläutert.

Es zeigen:
- Fig. 1: einen Längsschnitt des erfindungsgemäßen Dentalimplantats entlang einer Längsachse eines im Kieferknochen eines Patienten verankerbaren Basiskörpers, sowie
- Fig. 2: einen Querschnitt senkrecht zur Längsachse des Basiskörpers entlang der in Fig. 1 gekennzeichneten Schnittlinie II zur Darstellung der Verdrehsicherung.

Die Figuren sind lediglich schematischer Natur und dienen nur dem Verständnis der Erfindung. Die gleichen Elemente werden mit denselben Bezugszeichen versehen.

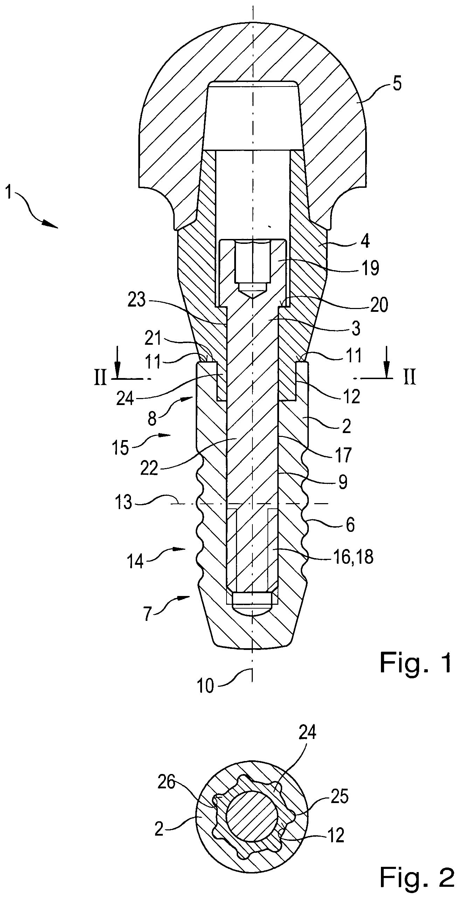

Die Fig. 1 zeigt ein Dentalimplantat 1 mit einem Basiskörper 2 und mit einem Implantataufbau 4, die mittels einer Schraube 3 aneinander befestigt sind. An dem Implantataufbau 4 kann wiederum auf bekannte Weise ein Zahnersatz 5 befestigt werden, beispielsweise durch Aufzementieren einer Zahnkrone.

Der Basiskörper 2 ist im Wesentlichen zylinderförmig ausgestaltet. Zur Verankerung in einem Kieferknochen (hier nicht dargestellt) weist der Basiskörper 2 ein Außengewinde 6 auf, das in eine Mantelfläche des Basiskörpers 2 eingebracht ist. Über dieses Außengewinde 6 wird der Basiskörper 2 in den Kieferknochen eingeschraubt. Die Mantelfläche läuft dabei zu dem, in dem Kieferknochen eingeschraubten, unteren Endbereich 7 des Basiskörpers 2 hin konisch zu, wodurch das Einschrauben des Basiskörpers 2 in den Kieferknochen erleichtert wird. Vom, vom Kieferknochen abgewandten, dem unteren Endbereich 7 gegenüberliegenden, oberen Endbereich 8 des Basiskörpers 2 her ist in den Basiskörper 2 eine Sacklochbohrung 9 eingebracht. Die Sacklochbohrung 9 ist im Basiskörper 2 mittig angeordnet und verläuft entlang der sich vom oberen Endbereich 8 bis zum unteren Endbereich 7 erstreckenden Längsachse 10 des Basiskörpers 2 bis in den unteren Endbereich 8 hinein. Die Längsachse 10 ist im Basiskörper somit koaxial zur Mittelachse der Sacklochbohrung 9.

Weiterhin am oberen Endbereich 8 des Basiskörpers 2 angeordnet ist eine ebene Auflagefläche 11 des Basiskörpers 2, die im Wesentlichen senkrecht (90°) zur Längsachse 10 verläuft, relativ zu der Längsachse 10. Zwischen der Sacklochbohrung 9 und der Auflagefläche 11 ist eine Aussparung 12 in dem Basiskörper 2 vorgesehen, die mit einem Gegenprofil eines am Implantataufbau 4 angebrachten Fortsatzes 24 zur Verdrehsicherung zusammenwirkt, wie weiter unten ausführlicher beschrieben ist. Die Auflagefläche 11 ist dabei der am weitesten nach oben, zum Implantataufbau 4 hin, in Richtung der Längsachse 10, hinausragende Abschnitt des Basiskörpers 2 und bildet somit eine Stirnseite des Basiskörpers 2.

Der Abstand in Richtung der Längsachse 10 des Basiskörpers 2 zwischen der Auflagefläche 11 und der dieser gegenüberliegenden, unteren Stirnseite des Endabschnittes 7, wird nachfolgend als "Länge" des Basiskörpers 10 bezeichnet. Diese Länge ist mittels einer imaginären Teilungsebene 13 in zwei gleich lange Hälften 14, 15 geteilt. In einer dem Kieferknochen zugewandten, unteren Hälfte 14 des Basiskörpers 2 ist ein Innengewinde 16 in die Sacklochbohrung 9 eingebracht. Die untere Hälfte 14 und die obere Hälfte 15 erstrecken sich demnach jeweils über die Hälfte der Länge des Basiskörpers 2. Die Sacklochbohrung 9 ragt dabei soweit in die untere Hälfte 14 hinein, dass die Länge der Sacklochbohrung 9 mindestens 80 %, besonders bevorzugt mindestens 90 %, ganz besonders bevorzugt mindestens 95 % der Gesamtlänge des Basiskörpers 2 beträgt. Das Innengewinde 16 selbst ist an einer dem unteren Endbereich 7 zugewandten Ende der Sacklochbohrung 9 angebracht und ist ausschließlich in der unteren Hälfte 14 des Basiskörpers 2 ausgebildet. Im Anschluss an das Innengewinde 16, in Richtung des Implantataufbaus 4, noch in der unteren Hälfte 14 des Basiskörpers 2, geht das Innengewinde 16 in eine Passbohrung 17 der Sacklochbohrung 9 über. Diese Passbohrung 17 erstreckt sich durchgehend bis zum oberen Endbereich des Basiskörpers 2, wo die Sacklochbohrung 9 in die Aussparung 12 übergeht. Die Passbohrung 17 ist länger als das Innengewinde 16.

Zur Befestigung des Implantataufbaus 4 wird die Schraube 3 soweit in die Sacklochbohrung 9 hineingesteckt, bis der Gewindeabschnitt 18 der Schraube 3 in das Innengewinde 16 eingeschraubt werden kann. Die Schraube 3 ist im zusammengebauten Zustand so weit in das Innengewinde 16 eingeschraubt, dass der Schraubenkopf 19 an einer Schraubenkopfauflage 20 des Implantataufbaus 4 anliegt. Der Implantataufbau 4, der mit einer Kontaktfläche 21 in diesem zusammengebauten Zustand an der Auflagefläche 11 des Basiskörpers 2 anliegt, wird durch das Anziehen der Schraube 3 in diesem Bereich fest gegen den Basiskörper 2 gepresst. Somit kommt es zu einem Druckspannungszustand in diesem Auflagebereich zwischen der Auflagefläche 11 und der Kontaktfläche 21.

In diesem zusammengebauten Zustand ist die Schraube 3 mit ihrem Gewindeabschnitt 18 vollständig in das Innengewinde 16 eingeschraubt. Somit greift der Gewindeabschnitt 18 vollständig in das Innengewinde 16 ein. Da sich das Innengewinde 16 vollständig in der unteren Hälfte 14 des Basiskörpers 2 befindet, befindet sich somit auch der in Eingriff befindliche Teil der Schraube 3 vollständig in der unteren Hälfte 14 des Basiskörpers 2. An den Gewindeabschnitt 18 der Schraube 3 schließt ein glatter Schaft 22 an, der derart mit der Passbohrung 17 des Basiskörpers 2 zusammenpasst, dass eine Spielpassung mit sehr geringem Spiel besteht. Vorzugsweise ist die Passung zwischen dem Schaft 22 und der Passbohrung 17 sogar als Übergangspassung ausgeführt, so dass ein leichter Druck zum Einschieben der Schraube 3 in die Sacklochbohrung 9 ausgeübt werden muss. Die Schraube 3 lässt sich somit gerade noch in der Passbohrung 17, in Richtung der Längsachse 10 verschieben, liegt jedoch mit der Außenfläche des Schafts 22 zumindest teilweise an der Innenfläche der Passbohrung 17 an. Die Mittelachse der Schraube 3 liegt im zusammengebauten Zustand vorzugsweise koaxial zu der Längsachse 10 des Basiskörpers 2.

Der Schaft 22 erstreckt sich von der die Schraubenkopfauflage 20 berührenden Seite des Schraubenkopfes 19 bis zum Gewindeabschnitt 18. Auch in dem Implantataufbau 4, in dem sich der Schaft 22 teilweise erstreckt, ist dieser in einer Passbohrung 23 des Implantataufbaus 4 geführt. Die Passung zwischen dem Schaft 22 und der Passbohrung 23 im Implantataufbau 4 ist dabei auch als Spielpassung mit sehr geringem Spiel ausgeführt. Somit kommt es sowohl zu einer Abstützung des Implantataufbaus 4 als auch des Basiskörpers 2 in Querrichtung der Mittelachse der Schraube 3 und, da diese Schraubenachse koaxial zur Längsachse 10 des Basiskörpers 2 verläuft, auch zu einer Abstützung in Querrichtung der Längsachse 10 des Basiskörpers 2.

Der Implantataufbau 4 weist zur radialen Fixierung des Implantataufbaus 4 relativ zum Basiskörper 2 einen Fortsatz 24 auf, der in die Aussparung 12 des Basiskörpers 2 einsteckbar ist. Im zusammengebauten Zustand kommt es durch das Einstecken zu einer radialen Lagerung beider Bauteile und somit zu einer Abstützung in Querrichtung der Längsachse 10.

Wie in Fig. 2 besonders gut ersichtlich ist, weist der Fortsatz 24 eine bestimmte Positivform auf, die in eine Negativform der Aussparung 12 im zusammengebauten Zustand eingeschoben ist. Somit ist eine Verdrehsicherung des Basiskörpers 2 relativ zum Implantataufbau 4, um die Längsachse 10 herum, gewährleistet. Das Profil des Fortsatzes 24 verläuft dabei im Querschnitt, also quer zur Längsachse 10, im Wesentlichen ringförmig, welche Ringform an ihrer, der Aussparung 12 zugewandten Außenseite längs verlaufende Auswölbungen 25 aufweist. Diese Auswölbungen 25 sind äquidistant um den, durch die Längsachse 10 festgelegten Mittelpunkt des Fortsatzes 24 herum an der Außenseite angeordnet. In diesem Ausführungsbeispiel sind sieben Auswölbungen 25 gleichmäßig um den Mittelpunkt verteilt, es ist jedoch auch möglich, eine andere Anzahl größer oder kleiner sieben der parallel zueinander angeordneten Auswölbungen 25 zu verwenden. Die der Außenseite des Fortsatzes 24 zugewandte Seite der Aussparung 12, welche an die Sacklochbohrung 9 und die Auflagefläche 11 anschließt, erstreckt sich ebenfalls, zumindest teilweise parallel zur Längsachse 10 und weist eine zu dem Profil des Fortsatzes 24 passende Negativform auf, so dass die Auswölbungen 25 in Rillen 26 der Aussparung 12 eingreifen. Durch den Eingriff kommt es somit zu einer Abstützung des Fortsatzes 24 an der Aussparung 12. Eine Verdrehung des Basiskörpers 2 relativ zum Implantataufbau 4 ist daher nicht möglich.

Der Implantataufbau 4, der im Wesentlichen als Hohlzylinder ausgestaltet ist, weist an einem, dem Basiskörper 2 abgewandten Ende einen üblichen Zahnersatz 5 auf, der im zusammengebauten Zustand auf den Implantataufbau 4 zementiert ist.

Der Basiskörper 2 besteht in diesem Ausführungsbeispiel vollständig aus Keramik, nämlich aus Zirkonoxid, wodurch auch das Innengewinde 16 an seinen, mit dem Gewindeabschnitt 18 der Schraube 3 in Eingriff befindlichen Gewindeflanken dieses keramische Material aufweist. Auch der Implantataufbau 4 ist vollständig aus keramischem Material, nämlich Zirkonoxid aufgebaut. Sowohl der Basiskörper 2 als auch der Implantataufbau 4 sind im Pulverspritzgießverfahren hergestellt, vorzugsweise im Ceramic Injection Molding (CIM)-Verfahren.

Die Schraube 3, oder zumindest der Schaft 22 der Schraube 3, ist aus einer Stahllegierung, besonders bevorzugt einer Titanlegierung, hergestellt.

Für den Zusammenbau des Dentalimplantats 1 wird der Basiskörper 2 zunächst in den Kieferknochen eingeschraubt. Ist dieser ausreichend stark verankert, vorzugsweise bereits an dem Knochen eingewachsen, so wird anschließend der Implantataufbau 4 durch Eindrehen der Schraube 3 in die Bohrungen des Implantataufbaus 4 und des Basiskörpers befestigt. Die Schraube 3 wird dabei so auf Zug belastet, dass der Implantataufbau 4 und der Basiskörper 2 fest gegeneinander angedrückt werden. Durch die senkrecht zur Längsachse 10 ausgestaltete Auflagefläche 12 und die daran anliegende Kontaktfläche 21 ist eine flächige Auflage des Implantataufbaus 4 an dem Basiskörper 2 gewährleistet. Im Auflagebereich kommt es daher überwiegend zu Druckspannungen entlang der Längsachse 10 und parallel zur Mittelachse der Schraube 3. Anschließend an das Festziehen der Schraube 3 wird dann der als Krone oder Brücke ausgeführte Zahnersatz 5 auf den Implantataufbau 4 aufzementiert.

## Patentansprüche

1. Dentalimplantat (1) mit einem in einem Kieferknochen verankerbaren Basiskörper (2) aus Keramik, einem an dem Basiskörper (2) befestigbaren Implantataufbau (4), und mit einer Schraube (3) zur Befestigung des Implantataufbaus (4) an dem Basiskörper (2), wobei in einem zusammengebauten Zustand des Dentalimplantats (1) ein Gewindeabschnitt (18) der Schraube (3) in ein, in einem Sackloch (9) des Basiskörpers (2) ausgebildetes Innengewinde (16) eingreift, und wobei der Implantataufbau (4) durch die Schraube (3) gegen den Basiskörper (2) gedrückt ist, **dadurch gekennzeichnet, dass** der Eingriff zwischen dem Gewindeabschnitt (18) der Schraube (3) und dem Innengewinde (16) des Sackloches (9) ausschliesslich in einer dem Implantataufbau (4) abgewandten, unteren Hälfte (14) des Basiskörpers (2) besteht, wobei die Sacklochbohrung (9), an das Innengewinde (16) in Richtung des Implantataufbaus (4) anschliessend, als Passbohrung (17) ausgeführt ist, wobei zwischen der Passbohrung (17) und einem Schaft (22) der eingesetzten Schraube (3) eine Spielpassung mit sehr geringem Spiel oder eine Übergangspassung besteht, sodass eine Abstützung in Querrichtung zur Längsachse (10) der Schraube (3) bereitgestellt wird.

2. Dentalimplantat (1) nach Anspruch 1, **dadurch gekennzeichnet, dass** sich das Innengewinde (16) über seine gesamte Länge ausschliesslich in der unteren Hälfte (14) des Basiskörpers (2) erstreckt.

3. Dentalimplantat (1) nach einem der vorherigen Ansprüche, **dadurch gekennzeichnet, dass** die Länge des Sackloches (9) mindestens 80 %, besonders bevorzugt mindestens 90 % der Gesamtlänge des Basiskörpers (2) beträgt.

4. Dentalimplantat (1) nach einem der vorherigen Ansprüche, **dadurch gekennzeichnet, dass** die Länge der Passbohrung (17) grösser als die Länge des Innengewindes (16) ist.

5. Dentalimplantat (1) nach einem der vorherigen Ansprüche, **dadurch gekennzeichnet, dass** der Implantataufbau (4) an einer im Wesentlichen senkrecht zu der Längsachse (10) der Schraube (3) verlaufenden Auflagefläche (11) an dem Basiskörper (2) aufliegt.

6. Dentalimplantat (1) nach einem der vorherigen Ansprüche, **dadurch gekennzeichnet, dass** der Implantataufbau (4) einen Fortsatz (24) aufweist, der im zusammengebauten Zustand des Dentalimplantats (1) in eine Aussparung (12) des Basiskörpers (2) eingesteckt ist.

7. Dentalimplantat (1) nach einem der vorherigen Ansprüche, **dadurch gekennzeichnet, dass** zwischen dem Implantataufbau (4) und dem Basiskörper (2) ein verdrehsicherer Formschluss besteht.

8. Dentalimplantat (1) nach einem der vorherigen Ansprüche, **dadurch gekennzeichnet, dass** sowohl der Implantataufbau (4) als auch der Basiskörper (2) aus keramischem Material, vorzugsweise Zirkonoxid, hergestellt sind.

9. Dentalimplantat (1) nach einem der vorherigen Ansprüche, **dadurch gekennzeichnet, dass** der Implantataufbau (4) und/oder der Basiskörper (2) mittels Pulverspritzgiessverfahren hergestellt sind/ist.

## Claims

1. A dental implant (1) with a ceramic base body (2) that can be anchored in a jawbone, an implant abutment (4) that can be attached to the base body (2), and a screw (3) for securing the implant abutment (4) to the base body (2), wherein, in an assembled state of the dental implant (1), a threaded portion (18) of the screw (3) engages in an internal thread (16) that is formed in a blind bore (9) of the base body (2), and wherein the implant abutment (4) is pressed by the screw (3) against the base body (2), **characterized in that** the engagement between the threaded portion (18) of the screw (3) and the internal thread (16) of the blind bore (9) takes place exclusively in a lower half (14) of the base body (2), which lower half (14) faces away from the implant abutment (4), wherein the blind bore (9) adjoining the internal thread (16) in a direction towards the implant abutment (4)is embodied as a fit bore, and wherein a clearance fit with very little clearance or a transition fit exists between the fit bore (17) and a shaft (22) of the inserted screw (3), so that a support is provided in the transverse direction with respect to the longitudinal axis (10) of the screw (3).

2. The dental implant (1) as set forth in claim 1, **characterized in that** the internal thread (16) extends over its entire length exclusively in the lower half (14) of the base body (2).

3. The dental implant (1) as set forth in any one of the preceding claims, **characterized in that** the length of the blind bore (9) is at least 80%, particularly preferably at least 90%, of the total length of the base body (2).

4. The dental implant (1) as set forth in any one of the preceding claims, **characterized in that** the length of the fit bore (17) is greater than the length of the internal thread (16).

5. The dental implant (1) as set forth in any one of the preceding claims, **characterized in that** the implant abutment (4) rests on a support surface (11) on the base body (2), which support surface (11) extends substantially perpendicularly with respect to the longitudinal axis (10) of the screw (3).

6. The dental implant (1) as set forth in any one of the preceding claims, **characterized in that** the implant abutment (4) has an extension (24) which, in the assembled state of the dental implant (1), is inserted into a recess (12) of the base body (2).

7. The dental implant (1) as set forth in any one of the preceding claims, **characterized in that** a torsion-proof positive fit exists between the implant abutment (4) and the base body (2).

8. The dental implant (1) as set forth in any one of the preceding claims, **characterized in that** both the implant abutment (4) and the base body (2) are made of ceramic material, preferably zirconium oxide.

9. The dental implant (1) as set forth in any one of the preceding claims, **characterized in that** the implant abutment (4) and/or the base body (2) are/is manufactured by means of powder injection molding processes.

## Revendications

1. Implant dentaire (1) comportant un corps de base (2) en céramique pouvant être ancré dans un os de mâchoire, une structure d'implant (4) pouvant être fixée au corps de base (2) et une vis (3) pour fixer la structure d'implant (4) au corps de base (2), dans lequel, à l'état assemblé de l'implant dentaire, une partie filetée (18) de la vis (3) s'engage dans un taraudage (16) réalisé dans un trou borgne (9) du corps de base (2), et la structure d'implant (2) est pressée contre le corps de base (2) par la vis (3), **caractérisé en ce que** l'engagement entre la partie filetée (18) de la vis (3) et le taraudage (16) du trou borgne (9) se situe exclusivement dans une moitié inférieure (14) du corps de base (2) détournée de la structure d'implant (4), le trou borgne (9) étant conçu comme un alésage de positionnement de manière adjacente au taraudage (16) en direction de la structure d'implant (4), un ajustement avec jeu avec un très faible jeu ou un ajustement de transition étant prévu entre l'alésage de positionnement (17) et une tige (22) de la vis insérée de façon à fournir un appui dans la direction transversale (10) par rapport à l'axe longitudinal (10) de la vis (3).

2. Implant dentaire (1) selon la revendication 1, **caractérisé en ce que** le taraudage (16) s'étend sur toute sa longueur exclusivement dans la moitié inférieure (14) du corps de base (2).

3. Implant dentaire (1) selon l'une des revendications précédentes, **caractérisé en ce que** la longueur du trou borgne (9) est égale à au moins 80 %, en particulier au moins 90 % de la longueur totale du corps de base (2).

4. Implant dentaire (1) selon l'une des revendications précédentes, **caractérisé en ce que** la longueur de l'alésage de positionnement (17) est supérieure à la longueur du taraudage (16) .

5. Implant dentaire (1) selon l'une des revendications précédentes, **caractérisé en ce que** la structure d'implant (4) est en appui sur une surface d'appui (11) s'étendant de manière sensiblement perpendiculaire à l'axe longitudinal (10) de la vis (3), au niveau du corps de base (2).

6. Implant dentaire (1) selon l'une des revendications précédentes, **caractérisé en ce que** la structure d'implant (4) comporte une saillie (24) qui, à l'état assemblé de l'implant dentaire (1), est insérée dans un évidement (12) du corps de base (2).

7. Implant dentaire (1) selon l'une des revendications précédentes, **caractérisé en ce qu'**une liaison par complémentarité de formes non rotative est prévue entre la structure d'implant (4) et le corps de base (2).

8. Implant dentaire (1) selon l'une des revendications précédentes, **caractérisé en ce qu'**aussi bien la structure d'implant (4) que le corps de base (2) sont fabriqués à partir d'une matière céramique, de préférence d'oxyde de zircone.

9. Implant dentaire (1) selon l'une des revendications précédentes, **caractérisé en ce que** la structure d'implant (4) et/ou le corps de base (2) est fabriqué(e) par un procédé de moulage par injection de poudre.
